# EUROPÄISCHE PATENTSCHRIFT

(11) **EP 2 496 168 B1**
(45) Veröffentlichungstag und Bekanntmachung des Hinweises auf die Patenterteilung: **19.03.2014**
(21) Anmeldenummer: 10779203.8
(22) Anmeldetag: 04.11.2010
(51) Int. Cl.: A61C 8/00, A61F 2/36

(54) **KERAMISCHES IMPLANTAT**
CERAMIC IMPLANT
IMPLANT CÉRAMIQUE

(30) Priorität: 04.11.2009 CH 17002009
(43) Veröffentlichungstag der Anmeldung: 12.09.2012
(73) Patentinhaber: New Dent AG, 4702 Oensingen (CH)
(72) Erfinder: PERLER, Peter, CH-2562 Port (CH); SCHWENTER, Peter, CH-4563 Gerlafingen (CH)
(74) Vertreter: Frei Patent Attorneys
(86) Internationale Anmeldenummer: PCT/CH2010/000275
(87) Internationale Veröffentlichungsnummer: WO 2011/054119

(56) Entgegenhaltungen:
- WO-A-03/013383
- WO-A-03/045268
- DE-A1-102006 011 629
- US-A- 5 947 735
- US-A1- 2009 035 723

## Beschreibung

Die Erfindung betrifft ein Keramik-Implantat, insbesondere ein Dentalimplantat, sowie ein Verfahren zur Herstellung eines solchen Implantats.

Aus der Medizinaltechnik sind keramische Implantate bekannt. Insbesondere keramische Dentalimplantate erfreuen sich einer wachsenden Beliebtheit, unter anderem wegen ihrer Eigenschaft, nur wenig sichtbares Licht zu absorbieren, aufgrund welcher sie weiss erscheinen können.

Bei Implantaten soll die mit dem Knochen in Berührung kommende Oberfläche mit dem Knochen verheilen, d.h. in einem Osseointegrationsprozess soll der Knochen in Oberflächenrauheiten des Implantats hineinwachsen, sodass das Implantat mit dem Knochen solid strukturell und auch funktionell verbunden ist.

Zum Zwecke der Förderung der Osseointegration ist sowohl für keramische als auch für metallische Implantate ein bekanntes Vorgehen, den zum Einwachsen in den Knochen bestimmten Bereich mittels eines abtragenden Verfahrens (insbesondere Ätzen, Sandstrahlen, Wasserstrahlen, bspw. mit abrasiv wirkenden Partikeln im Wasser) oder mittels eines auftragenden Verfahrens (siehe bspw. WO 2005/027771) an der Oberfläche so zu modifizieren, dass eine gewünschte Oberflächenrauheit entsteht.

Die DE 10 2006 011 629 schlägt vor, die Oberfläche eines Zirkondioxid-Implantates mittels eines Laserstrahls zu modifizieren. Durch den Laserstrahl werden Linienstrukturen in den Zahnwurzelbereich des Keramikimplantats erzeugt.

Die Schrift US 5,947,735 betrifft ein selbstschneidendes metallisches Dentalimplantat, bei dem Oberflächenbereiche behandelt sind, um sie mit einer Oberflächenrauhigkeit zu versehen. Mindestens eine vorstehende Schneidekante ist dabei jedoch nicht aufgerauht, damit sie durch das aufrauende Verfahren nicht stumpf wird.

Die Schrift WO 03/013383 zeigt ein Implantat aus Titan oder Keramik, das durch Strählen mit Rillen versehen wird, die in einem Winkel zur Implantatachse verlaufen. Das Implantat kann gestuft sein, wobei die verschiedenen Stufen unterschiedliche Rillenstrukturen aufweisen können.

Nachteilig an solchen Verfahren - angewandt auf keramische Implantate - ist, dass diese die besonderen Materialeigenschaften von keramischen Werkstoffen ausser Acht lassen. Insbesondere steigt die Anfälligkeit des Implantates auf einen Sprödbruch durch die Oberflächenbehandlung, weil durch die Behandlung minimale Oberflächendefekte entstehen. Diese können die Bruchfestigkeit des Implantats markant herabsetzen. Dies ist insbesondere bei einem lasttragenden Implantat wie beispielsweise einem Dentalimplantat von Bedeutung.

Es ist eine Aufgabe der Erfindung hier Abhilfe zu schaffen und ein Implantat sowie ein Verfahren zur Herstellung eines Implantats zur Verfügung zu stellen, welche die Nachteile des Standes der Technik überwinden und welche insbesondere die Bruchfestigkeit von Keramikimplantaten erhöhen ohne die Fähigkeit zur Osseointegration signifikant zu beeinträchtigen.

Diese Aufgabe wird gelöst durch das Implantat sowie das Verfahren, wie sie in den Patentansprüchen definiert sind.

Gemäss einem Aspekt der Erfindung wird ein keramisches Implantat, insbesondere ein Dentalimplantat zur Verfügung gestellt, welches einen keramischen, enossalen Oberflächenbereich aufweist, d.h. einen Bereich, der zur Einbettung ins Knochengewebe bestimmt ist und aus einem keramischen Werkstoff gefertigt ist. Dieser Oberflächenbereich weist mindestens eine erste Region mit einer Oberflächenmodifikation, in welcher die Oberfläche aufgerauht oder porös ist, und mindestens eine zweite Region, in der die Oberfläche nicht aufgerauht oder porös ist, auf.

Insbesondere weist derjenige axiale Teilbereich des keramischen Implantats, welcher die erste Region/die ersten Regionen aufweist, auch die zweite(n) Region(en) oder Anteile davon auf. Das heisst die zweiten Regionen beschränken sich nicht auf einen proximalen und/oder distalen Endbereich, sondern erstrecken sich zu axialen Positionen, an denen und/oder beidseitig von denen auch die erste(n) Region(en) vorhanden ist/sind.

Im die erste Region/die ersten Regionen umfassenden axialen Teilbereich auf (d.h. i.A. ein Bereich des Implantats mit in Bezug auf eine Längs- und/oder Insertionsachse axialen Position in einem bestimmten Grössenbereich, bspw. einen zwischen einem distalen Endbereich und dem nicht enossalen Bereich liegenden Zwischenbereich) sind also sowohl aufgerauhte oder poröse als auch nicht aufgerauhte Oberflächenanteile vorhanden sind. Mit anderen Worten erstreckt/erstrecken sich sowohl die erste(n) als auch die zweite(n) Region(en) über mindestens einen gemeinsamen axialen Teilbereich des enossalen Bereichs. Die erste(n) und die zweite(n) Region(en) greifen ineinander. Dieser axiale Teilbereich ist beispielsweise mindestens ein Teil des mit einem Gewinde versehenen Bereichs.

Dieses Vorgehen ermöglicht, dass gezielt an denjenigen Stellen, an denen der tragende Querschnitt besonders klein ist oder an denen aus anderen Gründen die Kräfte besonders gross sind, die Oberfläche nicht modifiziert und das Implantat folglich nicht geschwächt wird. Das Implantat kann also so ausgebildet sein, dass es die die Oberfläche der zweiten Regionen im Durchschnitt einen kleineren Abstand zur Längsachse hat als die Oberfläche der ersten Regionen. Es ist durch diese einfache Massnahme ein gezieltes Verstärken des Keramikimplantats im Vergleich zum Stand der Technik ermöglicht.

Bevorzugt sind die erste(n) und die zweite(n) Region(en) ineinander verschachtelt in dem Sinn, dass entlang einer Umfangsrichtung und/oder insbesondere in axialer Richtung verlaufenden Linie erste und zweite Regionen mehrfach ablösen; d.h. die ersten und zweiten Regionen sind nicht bloss ein proximaler und ein distaler Bereich. Die ersten Regionen erstrecken sich beispielsweise durch die zweiten Regionen unterbrochen über den ganzen enossalen Bereich, mit der möglichen Ausnahme einer distalen Endfläche oder eines distalen Endbereichs. In allen Ausführungsbeispielen kann die erste Region ein einziges zusammehängendes Gebilde sein. Dies kann beispielsweise der Fall sein, wenn das Implantat ein Gewinde aufweist; die erste Region kann dann zusammenhängend entlang der sich schraubenartig um den enossalen Bereich windenden Gewindespitzen verlaufen (mit dem Begriff 'Gewindespitzen' wird keine Aussage über die genaue Querschnittsform des Gewindes gemacht, d.h. auch Gewindespitzen mit im Querschnitt abgeflachte oder abgerundete Formen sind mit gemeint). Es ist aber auch möglich, dass die ersten Regionen eine Mehrzahl von voneinander beabstandeten Regionen sind. Dasselbe gilt auch für die zweite(n) Region(en). Nachfolgend ist jeweils von den ersten und zweiten Regionen (im Plural) die Rede; dabei sind Ausführungsformen mit einer einzigen zusammenhängenden ersten bzw. zweiten Region mitgemeint.

Die ersten Regionen machen beispielsweise mindestens 20%, mindestens 30%, mindestens 40% oder mindestens 50% der Oberfläche des enossalen Bereichs und beispielsweise maximal 93%, maximal 90% oder maximal 85%.

Dabei weist die enossale Partie des Implantats Retentions- und/oder Drehsicherungsstrukturen auf, beispielsweise ein Gewinde und/oder eine Rippenstruktur. Die Erhebungen (bzw. im Falle eines Gewindes die mindestens eine schraubenartig umlaufende Erhebung) dieser Retentions- und/oder Drehsicherungsstrukturen ist/sind dabei mindestens teilweise als erste Regionen ausgestaltet und weist/weisen eine Aufrauung oder poröse Struktur auf, während zwischen dieser Erhebung/diesen Erhebungen vorhandene Vertiefungen als zweite Regionen ausgestaltet sind und keine bzw. eine geringere Aufrauung oder poröse Struktur aufweist/aufweisen.

Es kann also insbesondere vorgesehen sein, dass im genannten Teilbereich der Oberflächenverlauf entlang einer Linie in axialer Richtung oder eventuell in Umfangsrichtung nacheinander als eine Mehrzahl von Erhebungen (bspw. Gewindekuppen oder Drehsicherungskämmen) mit dazwischen angeordneten Vertiefungen erscheint. Eine Vertiefung zeichnet sich dadurch aus, dass der Implantatdurchmesser lokal am Ort der Vertiefung kleiner ist als am Ort der beiden benachbarten Erhebungen. Der Abstand zwischen den benachbarten Erhebungen und die Tiefe der Vertiefungen sind dabei durch ihre Funktion als Retentions- bzw. Drehsicherungsstrukturen gegeben, sie sind dadurch im Allgemeinen - bspw. um mindestens eine Grössenordnung - grösser als die entsprechenden Dimensionen der durch die Modifikation zugefügten Oberflächenrauhigkeiten.

Durch dieses Vorgehen gemäss der erwähnten bevorzugten Ausgestaltung wird eine von Oberflächendefekten im Bereich der Vertiefungen - also dort, wo die Materialstärke am geringsten ist - ausgehende Schwächung verhindert. Die Bruchlast kann durch die hier vorgeschlagenen Massnahmen gezielt erhöht werden, insbesondere bei Materialien, die zum sogenannten Sprödbruch neigen.

Wenn die Retentions- und/oder Drehsicherungsstrukturen als Gewinde ausgestaltet sind oder ein Gewinde aufweisen, ist eine Umgebung der Gewindespitzen (bspw. bis in die Gewindeflanken hinein) oberflächenmodifiziert, während die Oberflächenmodifikation nicht im Gewindegrund angewendet ist.

Das Vorgehen gemäss der Erfindung ist insbesondere bei keramischen Dentalimplantaten oder anderen keramischen Implantaten mit lasttragenden Funktionen vorteilhaft. Unter den Dentalimplanaten kommen die einteiligen und einstückigen Implantate in Frage, aber auch mehrstückige einteilige oder mehrteilige Implantate.

Als keramisches Material für das Implantat kommen Materialien auf Zirkonoxid-Basis oder auf Aluminiumoxid-Basis in Frage.

Das gezielte Versehen des enossalen Implantatoberflächenbereichs mit einer Oberflächenmodifikation kann beispielsweise mit einem der folgenden sequentiellen und/oder parallelen Verfahren geschehen:
- Laser-Abtragen mit einem fokussierten Lichtstrahl, insbesondere Laserlichtstrahl (sequentiell). Ein fokussierter Laserstrahl wird so der Oberfläche entlang geführt, dass er dort Material abträgt, wo die ersten Regionen entstehen sollen, also beispielsweise im Bereich der Gewindeflanken und -kuppen. Beispielsweise kann der Laser Gräben mit Inseln oder Graten dazwischen erzeugen, beispielsweise Gräben mit einer Tiefe von zwischen 10 µm und 50 µm, beispielsweise zwischen 10 µm und 30 µm, und einem Abstand von zwischen 25 µm und 300 µm. Dabei wird der Laser gezielt nur auf Stellen fokussiert, die zu den ersten Regionen gehören. Ergänzend oder alternativ kann die Laserstrahlfokussierung auch eine gezielt geringe Schärfentiefe aufweisen, so dass er nur in einer Tiefe abtragend wirken kann, die der radialen Position der Erhebungen entspricht, während er in grösserer Tiefe - also an der radialen Position der Vertiefungen des Implantats - bereits so defokussiert ist, dass er nicht mehr abtragen kann. Wenn diese Massnahme getroffen wird, kann der Laser auch in Bahnen geführt werden, die über die ganze Oberfläche der enossalen Partie des Implantats führen. Die zweiten Regionen können dann eventuell an ihrer Oberfläche aufgrund der weniger fokussierten Laserstrahlung geringfügige Modifikationen erfahren; diese sind aber in ihrer Wirkung klein im Vergleich zu den Oberflächenmodifikationen der ersten Regionen.
- Laser-Abtragen oder anderes aus einer bestimmten Richtung einwirkendes Verfahren (wie Sandstrahlen, Wasserstrahlen mit abrasiven Medien, etc.) so, dass die zweiten Regionen im Schatten liegen (sequentiell oder parallel). Beispielsweise kann der Strahl aus einem Winkel von ca. 45° zur Implantatachse auftreffen, wenn der Flankenwinkel des Gewindes 60° beträgt, wodurch der Gewindegrund im Schatten liegt. Generell wird bevorzugt der Auftreffwinkel als kleiner als der Flankenwinkel von Retentions- und/oder Drehsicherungsstrukturen gewählt, wodurch Vertiefungen dieser Strukturen im Kernschatten liegen, wobei auch grössere von 90° verschiedene Auftreffwinkel eine Wirkung haben, weil dadurch die Vertiefungen im Halbschatten liegen. Auch in dieser Ausführungsform kann ein Laserlichtstrahl fokussiert entlang von Bahnen geführt werden, die im Wesentlichen über die ganze enossale Partie führen; eine Ergänzung dieses Vorgehens mit einer Fokussierung mit einer limitierten Schärfentiefe ist möglich.
- Abtragende oder auftragende Verfahren unter Verwendung einer Maske. Eine Maske kann beispielsweise durch ein selektives Verfahren oder durch Rakelähnliche Techniken oder dergleichen in die Vertiefungen hinein aufgebracht werden. Entsprechende abtragende Verfahren können beispielsweise das Ätzen (parallel) oder aber - bei gegebener Beständigkeit der Maske - auch Laser-Abtragen, Sandstrahlen, Wasserstrahlen mit abrasiven Medien etc. (sequentiell oder parallel) verwendet sein. Ein Beispiel eines auftragenden Verfahrens findet man in WO 2004/017 857.

Auch Kombinationen dieser Verfahren, beispielsweise Laser-Abtragen mit einer anschliessenden Ätz-Nachbehandlung sind möglich.

Das 'Laser-Abtragen' wird im Allgemeinen mit einer Laserlichtquelle gemacht, wobei andere sehr starke Lichtquellen, bspw. Superlumineszenzlichtquellen oder Blitzlichtquellen nicht grundsätzlich auszuschliessen sind. Mit 'Lichtquelle' werden hier selbstverständlich nicht nur im sichtbaren Bereich emittierende Strahlungsquellen bezeichnet, sondern generell Quellen elektromagnetischer Strahlung, insbesondere im sichtbaren und infraroten Bereich, aber auch Quellen von Strahlung kürzerer Wellenlängen.

Implantate der erfindungsgemässen Art sind beispielsweise voll keramisch. Sie können beispielsweise aus einem Werkstoff hergestellt sein, welcher im Wesentlichen aus Zirkonoxid besteht, also bspw. zu mindestens 90% ZrO₂ aufweist und welchem zusätzlich andere Bestandteile, beispielsweise Yttriumoxid und/oder Hafniumoxid und/oder kleinere Mengen Aluminiumoxid und/oder Siliziumoxid und/oder weitere Oxide oder andere Bestandteile beigemischt sein können. Alternativ dazu kann der Werkstoff auch im Wesentlichen aus Aluminiumoxid oder einer Zirkonoxid-Aluminiumoxid-Mischung bestehen, eventuell ebenfalls Zusätzen. Auch andere keramische Werkstoffe sind denkbar.

Eine maximale Rauhtiefe der ersten Regionen beträgt beispielsweise zwischen 0.5 µm und 50 µm, bevorzugt zwischen 3 µm und 15 µm. Eine mittlere Rauhtiefe (mittlere Rauheit Ra) der ersten Regionen beträgt beispielsweise zwischen 0.2 µm und 50 µm oder zwischen 0.5 µm und 30 µm, bevorzugt zwischen 0.8 µm und 15 µm oder zwischen 1 µm und 10 µm. Bei Verwendung des Laser-Abtragverfahrens mit einem fokussierten Laserstrahl kann die Rauheit durch regelmässig angeordnete Gräben gebildet sein, wobei die Gräben eine Breite von beispielsweise zwischen 10 µm und 40 µm und einen mittleren Abstand von zwischen 15 µm und 60 µm haben können.

Eine maximale Rauhtiefe der zweiten Regionen beträgt beispielsweise maximal 3 µm, maximal 2 µm, maximal 1.5 µm oder maximal 1 µm; eine mittlere Rauhtiefe der zweiten Regionen beträgt beispielsweise 1 µm oder weniger, insbesondere weniger als 0.5 µm. Die mittlere Rauheit Ra der zweiten Regionen ist signifikant kleiner als die mittlere Rauheit der ersten Regionen und bevorzugt zwischen 0.02 und 0.5 µm, besondere bevorzugt weniger als 0.3 µm.

Der enossale Bereich kann zusätzlich zu den ersten und zweiten durch den keramischen Werkstoff gebildeten Oberflächenregionen noch weitere, durch andere Werkstoffe gebildete Oberflächenregionen aufweisen. So können beispielsweise thermoplastische Oberflächenregionen gemäss der WO 2004/017 857 vorhanden sein. Auf die Lehre dieser Schrift, insoweit sie lasttragende Implantate und insbesondere Dentalimplantate betrifft, wird hier ausdrücklich verwiesen.

Die Oberfläche des Implantats, der enossalen Partie, oder auch nur der ersten Regionen kann nachbehandelt werden, beispielsweise durch eine Silanisierung oder Hydroxilierung, wodurch die osseointegrative Wirkung verstärkt wird.

Nachfolgend werden Ausführungsbeispiele der Erfindung anhand von Figuren näher erläutert. In den Figuren bezeichnen gleiche Bezugszeichen gleiche oder analoge Elemente. Es zeigen:
- Figur 1 schematisch ein Dentalimplantat mit einem Gewinde als Retentionsstruktur;
- Figur 2 schematisch ein erstes Oberflächenmodifikationsverfahren;
- Figur 3 schematisch ein zweites Oberflächenmodifikationsverfahren:
- Figur 4 ebenfalls schematisch eine Maskentechnik für weitere Oberflächenmodifikationsverfahren;
- Figur 5 schematisch ein Dentalimplantat mit Drehsicherungsstrukturen;
- Figuren 6-9 elektronenmikroskopische Aufnahmen der Oberfläche eines erfindungsgemäss ausgeführten Dentalimplantates.

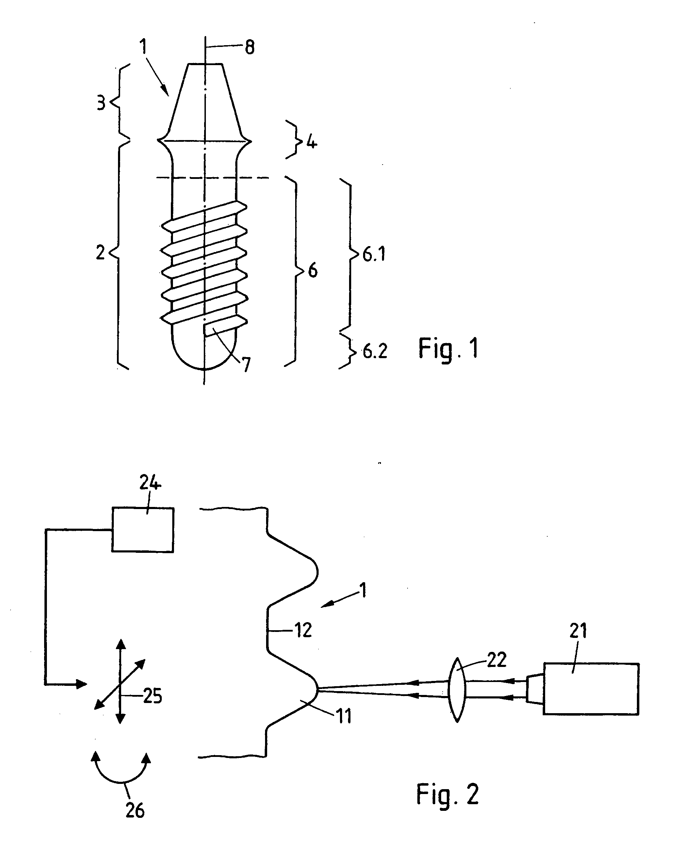

Das Implantat 1 gemäss **Figur 1** ist voll keramisch und beispielsweise im Wesentlichen aus Zirkonoxid gefertigt. Es weist eine Verankerungspartie 2 und eine mit diesem einstückige Aufbaupartie 3 zum Aufsetzen einer Krone (nicht gezeichnet) auf. Die Aufbaupartie kann auch dafür verwendet werden, während der Implantation ein Eindrehwerkzeug anzusetzen. Sie weist beispielsweise in an sich bekannter Art eine von der Zylindersymmetrie abweichende Struktur auf, damit die Krone bzw. das Eindrehwerkzeug drehfest mit dem Aufbau verbunden werden kann.

Im Bereich des Übergangs zwischen der Verankerungspartie 2 und der Aufbaubartie 3 weist das Implantat eine Aufweitung 4 auf, welche beispielsweise eine nach der Implantation auf das Zahnfleisch abgestützte und abdichtende Schulter bilden kann.

Ein distaler Bereich der Verankerungspartie 2, welcher einen Grossteil derselben ausmacht, bildet den enossalen Bereich 6, der nach der Implantation von Knochengewebe umgeben ist. Das Knochengewebe wächst nach der Implantation in Oberflächenstrukturen des enossalen Bereichs 6 ein. Im enossalen Bereich ist ein Gewinde 7 ausgebildet, das nach der Implantation die notwendige Primärstabilität gewährleistet und auch zur permanenten Verankerung des Implantats beiträgt. Der enossale Bereich 6 kann in einen ersten enossalen Teilbereich 6.1 und einen zweiten enossalen Teilbereich 6.2 (Endbereich) aufgeteilt werden. Der erste enossale Teilbereich 6.1 weist das Gewinde auf. Er muss sowohl lasttragend sein als auch Kräfte aufnehmend verankert sein. Er weist sowohl die ersten Regionen als auch die zweiten Regionen auf. Der zweite enossale Teilbereich 6.2 ist ein distaler Endbereich. Er kann eine aufgerauhte und/oder nicht aufgerauhte Oberläche aufweisen.

In Figur 1 ebenfalls eingezeichnet ist die Längsachse 8 (oder Insertionsachse), welche wie an sich bekannt eine Symmetrieachse (wobei die Symmetrie durch das Gewinde gebrochen wird) des enossalen Bereichs 6 oder gar des ganzen Implantats sein kann; das Implantat muss jedoch nicht notwendigerweise symmetrisch sein.

Gemäss der Erfindung weist nun der enossale Bereich 6 erste, modifizierte Oberflächenregionen mit einer gezielt beigebrachten Oberflächenrauheit und zweite, nicht modifizierte Oberflächenregionen auf, in welchen die Oberflächenrauheit gering, d.h. die Oberfläche glatt ist. Die Oberflächenbeschaffenheit der zweiten Oberflächenregionen wird im Allgemeinen durch das Verfahren bestimmt, mit welchem das Implantat in seine Form gebracht wird. Die modifizierten, ersten Oberflächenregionen haben eine im Vergleich zu den ersten Oberflächenregionen erhöhte Rauheit.

In der Ausführungsform gemäss Figur 1 umfassen die ersten Regionen die Kuppen und oberen Flankenbereiche des Gewindes; die zweiten Regionen den Gewindegrund. Teile des enossalen Bereichs, an denen das Gewinde nicht vorhanden ist - also hier das distale, abgerundete Ende sowie die Halspartie zwischen dem Gewinde und der Aufweitung 4 - können als zweite oder vorzugsweise mindestens teilweise als erste Regionen ausgestaltet sein.

**Figur 2** zeigt sehr schematisch ein Verfahren zum Applizieren der Oberflächenmodifikation in einem sequentiellen Verfahren mit einem fokussierten Lichtstrahl. Der Strahl einer geeigneten Laserlichtquelle 21 - bspw. eines Hochleistungs-Festkörperlasers, insbesondere eines Nd:YAG-Lasers oder eines Er:YAG-Lasers - wird auf die Oberfläche des vorgefertigten Implantates 1 fokussiert; der Durchmesser des Fokus kann an der engsten Stelle ca. 5 µm betragen. Durch eine Steuerung 24 kontrolliert werden das Implantat einerseits und die Laserlichtquelle 21 mit den Fokussierungsmitteln 22 andererseits relativ zueinander bewegt, was durch die eine translatorische Bewegung bzw. eine Rotationsbewegung darstellenden Pfeile 25, 26 angedeutet wird. Die Bewegung erfolgt so dass in der Summe in den ersten Regionen 11 eine Vielzahl von ca. 20 µm breiten und ca. 20 µm tiefen Gräben entsteht, wobei der Abstand von Graben zu Graben - von Grabenmitte zu Grabenmitte gemessen - ca. 40 µm betragen kann. Es entstehen also gerichtete Oberflächenrauhigkeiten. Die hat zusätzlich zu der Wirkung der bekannten zufällig ausgebildeten Oberflächenrauhigkeiten auch noch die Wirkung, dass sich Kollagenstrukturen des einwachsenden Knochengewebes nach den Gräben ausrichten können.

Beim Vorgehen gemäss Figur 2 können folgende Massnahmen allein oder in Kombination vorgesehen sein, damit die Oberflächenmodifikation nur in den ersten Regionen vorgenommen wird:
- Die Steuerung 24 steuert den Laser und die Bewegungsmittel so an, dass der Laser nur auf Oberflächenabschnitte einwirkt, die den ersten Regionen entsprechen bzw. zu diesen gehören. Zu diesem Zweck müssen die 3-dimensionale Struktur des Implantates 1 und dessen Position genau einprogrammiert sein.
- Der Laser ist so fokussiert, dass er eine vergleichsweise geringe Schärfentiefe hat bspw. von ca. 50-100 µm. Eine solche Fokussierung ist ohne Weiteres möglich. Die Achse des Implantats hat dann einen fixen Abstand zum Laser und zu den Fokussierungsmitteln, und der Lichtstrahl ist dann auf eine radiale Position des Implantats fokussiert, die der Position der Erhebungen entspricht. Dann kann der Laserlichtstrahl optional auch in Bahnen über die ganze Oberfläche der enossalen Partie geführt werden.

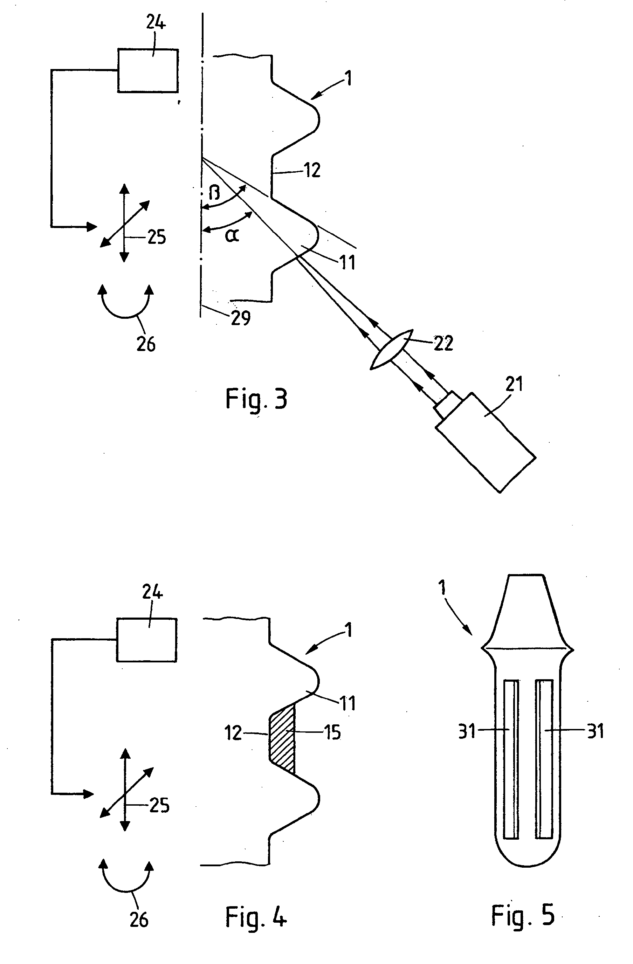

In **Figur 3** ist schematisch ein abtragendes Verfahren mit einem Laserstrahl gezeigt, bei welchem der Gewindegrund im Schatten bleibt, wodurch die Oberflächenrauhigkeit nur auf den Flanken und Spitzen entsteht. Damit der Gewindegrund 12 im Schatten ist, sollte der Winkel α der Einstrahlrichtung zur Implantatachse kleiner sein als der Winkel β der Gewindeflanken Implantatachse. In der Figur ist eine Achse 29 eingezeichnet, die zur Implantatachse (nicht gezeichnet) parallel ist. Beispielsweise kann der Winkel α ungefähr 45° betragen und der Flankenwinkel ca. 60°, wodurch auch der Winkel β ungefähr 60° beträgt.

Auch bei Einstrahlwinkeln α, die nicht ganz der obigen Bedingung entsprechen, sondern bei beispielsweise α≈β liegen, wird eine Wirkung erzielt.

Auch beim Vorgehen gemäss Figur 3 kann der Laserlichtstrahl optional in Bahnen über die ganze Oberfläche der enossalen Partie geführt werden, wodurch die genaue 3D-Geometrie des Implantats nicht erfasst und in die Steuerung einprogrammiert sein muss. Alternativ kann jedoch die Steuerung auch so programmiert sein, dass sie zur Ergänzung der Selektivität zusätzlich der Laser nur dann wirkt, wenn der Laserstrahl auf Oberflächenbereiche trifft, die zu den ersten Regionen gehören, oder dass der Laserstrahl nur über solche Oberflächenbereiche überhaupt geführt wird. Ein zu Figur 3 analoges Verfahren ist auch mit alternativen gerichteten abtragenden Verfahren möglich, wobei bei solchen Verfahren im Allgemeinen eine deutlich weniger ausgeprägte Fokussierung erfolgt, wodurch die Relativbewegung des Implantats zur Quelle des gerichteten abtragenden Mittels unter Umständen lediglich eine Schraubenbewegung oder gar nur eine Drehbewegung um die Achse sein muss.

**Figur 4** zeigt ebenfalls schematisch die Maskentechnik. Der Gewindegrund 12 ist durch eine Maske 15 abgedeckt. Ein paralleles abtragendes Verfahren (bspw. ätzen) oder ein auftragendes Verfahren kann dann an den nicht abgedeckten Stellen für die Oberflächenmodifikation sorgen. Die Maske kann aus einem geeigneten Resistmaterial sein, welche anschliessend an das Oberflächenmodifikationsverfahren wieder abgetragen werden kann.

**Figur 5** schliesslich zeigt ein Implantat 1, welches anstelle eines Gewindes axial verlaufende Drehsicherungsstrukturen 31 aufweist. Das Implantat kann zusätzliche, nicht gezeichnete Mittel zum Herstellen einer primären Stabilität aufweisen oder - abweichend von der dargestellten Ausführungsform - vom zweiteiligen Typ sein.

Auch bei einem Implantat mit Drehsicherungsstrukturen anstelle eines Gewindes kann das Prinzip angewandt werden, dass die ersten Oberflächenregionen im Bereich von Erhebungen angeordnet sind und die zweiten Oberflächenregionen im Bereich von dazwischen liegenden Vertiefungen. Dasselbe gilt für Implantate mit weiteren Retentionsstrukturen zusätzlich oder anstelle von Drehsicherungsstrukturen.

### Beispiel:

Ein Zirkonoxid-Dentalimplantat mit weniger als 10% Yttriumoxid wurde mit einem Gewinde versehen in an sich konventioneller Art gefertigt, indem ein Formling aus dem keramischen Werkstoff in einem Press-Sinterverfahren hergestellt wurde und anschliessend durch Schleifen in die gewünschte Form mit Gewinde gebracht. Als Keramikmaterial wird ein Yttrium-tabilisiertes, tetragonales, teilkristallines Zirkondioxid eingesetzt. Dabei erfiillt die verwendete Zirkondioxid Keramik die Norm ISO 13356:2008 zu "Implants for Surgery - Ceramic Materials based on yttria-stabilized tetragonal zirconia (Y-TZP)".

Anschliessend wurde mit einem Nd:YAG-Festkörperlaser (Wellenlänge 1064 nm) mit einer Ausgangsleistung von 20 Watt gepulst 5 bis 100 kHz, Arbeitsdistanz 100 mm und ein Fokus-Spot von 2 bis 10 µm, im Bereich der Gewindespitzen und Gewindeflanken die Oberfläche gezielt modifiziert. Zu diesem Zweck wurde der auf einen Fokus mit einem Durchmesser von 5 µm und mit einer geringen Schärfentiefe fokussierte Laserstrahl so über die Oberfläche geführt, dass eine Vielzahl von ca. 20 µm breiten und ca. 20 µm tiefen Gräben entstand, wobei der Abstand von Graben zu Graben ca. 40 µm betrug. Im Bereich des Gewindegrundes wurde keine Oberflächenmodifikation vorgenommen. Am resultierenden Dentalimplantat wurden Elektronenmikroskopiemessungen vorgenommen.

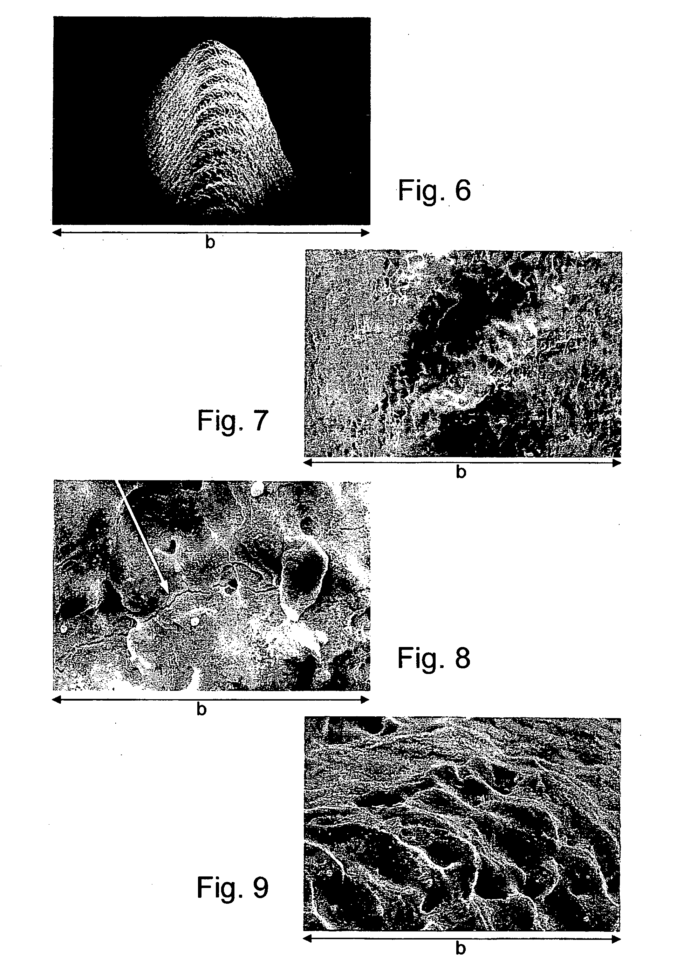

Figuren 6-9 zeigen eine Auswahl der entsprechenden Elektronenmikroskopaufnahmen. Die Aufnahmen wurden mit einem Elektronenstrahl von 20 kV gemacht.

**Figur 6** zeigt die Gewindespitze mit den gut sichtbaren Gräben in regelmässigen Abständen. Die ganze Breite *b* des in Figur 6 gezeigten Bereichs entspricht 700 µm. **Figur 7** zeigt ein Detail in senkrechter Ansicht und grösserer Auflösung (Breite *b*=200 µm); man sieht ein Tal zwischen zwei Erhebungen. **Figur 8** zeigt in noch grösserer Auflösung ein Detail des Tals; sehr gut erkennbar sind die für Oberflächenmodifikationsverfahren typischen Oberflächendefekte in Form von Rissen (heller Pfeil). **Figur 9** zeigt im gleichen Massstab wie Figur 9 (jeweils b= 50 µm), aber aus einem leicht schrägen Blickwinkel, eine Detail des Gewindegrundes ohne Oberflächenmodifikation; es sind kaum Oberflächendefekte sichtbar.

## Patentansprüche

1. Keramisches Implantat (1), insbesondere Dentalimplantat, mit einem keramischen, enossalen Oberflächenbereich einer enossalen Partie (6) des Implantats, wobei der keramische enossale Oberflächenbereich mindestens in einem axialen Teilbereich eine erste Region aufweist, in welcher die Oberfläche mit einer Oberflächenmodifikation in Form einer Aufrauhung oder porösen Struktur versehen ist, und der keramische enossale Oberflächenbereich in diesem Teilbereich mindestens eine zweite Region aufweist, in der die Oberfläche nicht oder nur in einem geringeren Ausmass mit der genannten Oberflächenmodifikation versehen ist, **dadurch gekennzeichnet, dass** die enossale Partie des Implantats Retentions- und/oder Drehsicherungsstrukturen (7, 31) aufweist und dass Erhebungen (11) dieser Retentions- und/oder Drehsicherungsstrukturen mindestens teilweise als erste Regionen ausgestaltet sind, während zwischen diesen Erhebungen vorhandene Vertiefungen (12) als zweite Regionen ausgestaltet sind.

2. Implantat nach Anspruch 1, **dadurch gekennzeichnet, dass** die mindestens eine zweite Region an Stellen vorhanden ist, an denen ein lasttragender Implantatquerschnitt kleiner ist als an Stellen mit der mindestens einen ersten Region.

3. Implantat nach Anspruch 1 oder 2, **dadurch gekennzeichnet, dass** die Retentions- und/oder Drehsicherungsstrukturen ein Gewinde (7) aufweisen, wobei Gewindespitzen und Bereiche von Gewindeflanken als erste Regionen ausgebildet sind und ein Gewindegrund als zweite Region ausgestaltet ist.

4. Implantat nach einem der vorangehenden Ansprüche, **dadurch gekennzeichnet, dass** es ein einteiliges und einstückiges Dentalimplantat (1) aus einem Werkstoff auf Zirkonoxid, Aluminiumoxid- oder Zirkonoxid- und Aluminiumoxidbasis hergestellt ist.

5. Implantat nach einem der vorangehenden Ansprüche, **dadurch gekennzeichnet, dass** eine maximale Rauhtiefe der ersten Regionen mindestens 3 µm beträgt und eine maximale Rauhtiefe der zweiten Regionen höchstens 2 µm beträgt.

6. Implantat nach einem der vorangehenden Ansprüche, **dadurch gekennzeichnet, dass** die Oberflächenmodifikation in Form einer Aufrauhung eine Mehrzahl von zueinander in regelmässigen Abständen angeordneten Gräben einer mittleren Tiefe von zwischen 0.5 µm und 35 µm, bevorzugt zwischen 3 µm und 35 µm, aufweist.

7. Implantat nach einem der vorangehenden Ansprüche, **dadurch gekennzeichnet, dass** die Oberflächenmodifikation in Form einer Aufrauhung mittels eines Laser-Abtragverfahrens beigebracht ist.

8. Verfahren zum Fertigen eines Implantats (1), insbesondere Dentalimplantats, wobei das Implantat einem keramischen, enossalen Oberflächenbereich einer enossalen Partie (6) aufweist, wobei in einem ersten Schritt das Implantat in die vorgesehene Form gebracht und in einem zweiten Schritt die Implantatoberfläche modifiziert wird, um eine Aufrauhung oder poröse Struktur zur Förderung der Osseiointegration zu erhalten, wobei im zweiten Schritt die Implantatoberfläche nur in ersten Regionen eines keramischen enossalen Oberflächenbereichs modifiziert wird, während zweite Regionen des keramischen enossalen Oberflächenbereichs nicht oder nur in einem geringeren Ausmass mit der Oberflächenmodifikation versehen werden, **dadurch gekennzeichnet, dass** das Implantat Retentions- und/oder Drehsicherungsstrukturen (7, 31) aufweist und dass Erhebungen (11) dieser Retentions- und/oder Drehsicherungsstrukturen mindestens teilweise als erste Regionen ausgestaltet werden, während zwischen diesen Erhebungen vorhandene Vertiefungen (12) als zweite Regionen ausgestaltet werden.

9. Verfahren nach Anspruch 8, **dadurch gekennzeichnet, dass** die Oberflächenmodifikation mit abtragenden Verfahren mittels eines gerichteten abtragendes Mittels vorgenommen wird.

10. Verfahren nach Anspruch 9, **dadurch gekennzeichnet, dass** das gerichtete abtragende Mittel ein Laserstrahl ist, wodurch die Oberflächenmodifikation mit einem fokussierten Laserstrahl vorgenommen wird.

11. Verfahren nach Anspruch 10, **dadurch gekennzeichnet, dass** der Laser so fokussiert wird, dass die Schärfentiefe kleiner ist als die Höhe charakteristischer Erhebungen im Bereich des enossalen Oberflächenbereichs.

12. Verfahren nach Anspruch 10 oder 11, **dadurch gekennzeichnet, dass** eine Relativbewegung des fokussierten Laserstrahls zum Implantat sowie die Leistung des Laserstrahls so gesteuert werden, dass der Laserstrahl nur an Stellen wesentlich abtragend wirkt, die zu den ersten Regionen gehören.

13. Verfahren nach einem der Ansprüche 9 bis 12, **dadurch gekennzeichnet, dass** die Richtung, aus der das Mittel einwirkt, in einem kleineren Winkel (α) zu einer Achse des Implantats steht als ein maximaler Steigungswinkel (β) zur Implantatachse von Erhebungen des enossalen Oberflächenbereichs, wodurch diese Erhebungen einen Schatten auf zwischen den Erhebungen liegende Vertiefungen werfen.

14. Verfahren nach einem der Ansprüche 8-13, **dadurch gekennzeichnet, dass** im zweiten Schritt die zweiten Regionen durch eine Maske (15) abgedeckt werden.

## Claims

1. A ceramic implant (1), in particular a dental implant, with a ceramic endosseous surface area of an endosseous portion (6) of the implant, wherein the ceramic endosseous surface area has, at least in one axial partial area, a first region, in which the surface is is provided with a surface modification in the form of a roughening or porous structure, and the ceramic endosseous surface area has, in this partial area, at least one second region, in which the surface is not provided with said-surface modification or is provided only to a lesser extent with said surface modification, **characterized in that** the endosseous portion of the implant has retention and/or anti-rotation structures (7, 31), and **in that** elevations (11) of these retention and/or anti-rotation structures are designed at least partially as first regions, whereas depressions (12) present between these elevations are designed as second regions.

2. The implant as claimed in claim 1, **characterized in that** the at least one second region is present at locations where a load-bearing implant cross section is smaller than at locations with the at least one first region.

3. The implant as claimed in claim 1 or 2, **characterized in that** the retention and/or anti-rotation structures have a thread (7), wherein thread crests and areas of thread flanks are designed as first regions, and a thread root is designed as second region.

4. The implant as claimed in one of the preceding claims, **characterized in that** it is a one-part and integral dental implant (1) produced from a material based on zirconia, alumina, or zirconia and alumina.

5. The implant as claimed in one of the preceding claims, **characterized in that** a maximum peak-to-vally height of the first regions is at least 3 µm, and a maximum peak-to-valley height of the second regions is at most 2 µm.

6. The implant as claimed in one of the preceding claims, **characterized in that** the surface modification in the form of a roughening has a plurality of trenches arranged at regular intervals to one another and having an average depth of between 0.5 µm and 35 µm, preferably between 3 µm and 35 µm.

7. The implant as claimed in one of the preceding claims, **characterized in that** the surface modification in the form of a roughening is brought about by means of a laser ablation method.

8. A method for producing an implant (1), in particular a dental implant, wherein the implant has a ceramic endosseous surface area of an endosseous portion (6), wherein, in a first step, the implant is brought to the intended shape and, in a second step, the implant surface is modified in order to obtain a roughening or porous structure for promoting osseointegration, wherein in the second step the implant surface is modified only in first regions of a ceramic endosseous surface area, whereas second regions of the ceramic endosseous surface area are not provided with the surface modification or are provided only to a lesser extent with the surface modification, **characterized in that** the endosseous portion of the implant has retention and/or anti-rotation structures (7, 31), and **in that** elevations (11) of these retention and/or anti-rotation structures are designed at least partially as first regions, whereas depressions (12) present between these elevations are designed as second regions.

9. The method as claimed in claim 8, **characterized in that** the surface modification is performed by ablation methods by means of a directed ablation medium.

10. The method as claimed in claim 9, **characterized in that** the directed ablation medium is a laser beam, as a result of which the surface modification is performed with a focussed laser beam.

11. The method as claimed in claim 10, **characterized in that** the laser is focussed such that the depth of field is less than the height of characteristic elevations in the area of the endosseous surface area.

12. The method as claimed in claim 10 or 11, **characterized in that** a movement of the focussed laser beam, relative to the implant, and the output of the laser beam are controlled such that the laser beam has a substantially ablating action only at locations belonging to the first regions.

13. The method as claimed in one of claims 9 through 12, **characterized in that** the direction from which the medium acts is at a smaller angle (α), to an axis of the implant, than a maximum angle of slope (β), to the implant axis, of elevations of the endosseous surface area, as a result of which these elevations cast a shadow on depressions lying between the elevations.

14. The method as claimed in one of claims 8-13, **characterized in that,** in the second step, the second regions are covered by a mask (15).

## Revendications

1. Implant céramique (1), notamment implant dentaire, présentant une zone céramique sur une partie (6) de la surface de l'implant, la zone céramique de la surface de l'implant présentant au moins dans une partie axiale une première région dans laquelle la surface est modifiée sous la forme d'une rugosité ou d'une structure poreuse, la zone céramique de la surface de l'implant présentant dans cette partie au moins une deuxième région dans laquelle la surface ne subit pas ladite modification ou ne la subit que dans une mesure réduite,
**caractérisé en ce que**
la partie de l'implant présente des structures (7, 31) de retenue et/ou de blocage de la rotation et
**en ce que** des reliefs (11) de ces structures de retenue et/ou de blocage de la rotation sont configurés au moins en partie comme premières régions tandis que des creux (12) prévus entre ces reliefs sont configurés comme deuxièmes régions.

2. Implant selon la revendication 1, **caractérisé en ce que** la ou les deuxièmes régions sont prévues en des emplacements auxquels la section transversale de l'implant reprenant les charges est plus petite qu'en des emplacements qui présentent la ou les premières régions.

3. Implant selon les revendications 1 ou 2, **caractérisé en ce que** les structures de retenue et/ou de blocage de la rotation présentent un filet (7), les sommets des filets et des parties des flancs des filets étant configurés comme premières régions et le fond des filets étant configuré comme deuxième région.

4. Implant selon l'une des revendications précédentes, **caractérisé en ce qu'**il est un implant dentaire (1) d'une seule pièce dont le matériau est à base d'oxyde de zirconium, d'oxyde d'aluminium ou d'oxyde de zirconium et d'oxyde d'aluminium.

5. Implant selon l'une des revendications précédentes, **caractérisé en ce que** la profondeur maximale des rugosités des premières régions est d'au moins 3 µm et la profondeur maximale des rugosités des deuxièmes régions est d'au plus 2 µm.

6. Implant selon l'une des revendications précédentes, **caractérisé en ce que** la modification de la surface qui présente la forme d'une rugosité présente plusieurs sillons disposés à distance mutuelle régulière et d'une profondeur moyenne comprise entre 0,5 µm et 35 µm et de préférence entre 3 µm et 35 µm.

7. Implant selon l'une des revendications précédentes, **caractérisé en ce que** la modification de la surface présente la forme d'une rugosité réalisée au moyen d'une opération d'enlèvement de matière par laser.

8. Procédé de fabrication d'un implant (1), notamment d'un implant dentaire, l'implant présentant une partie céramique dans une partie (6) de la surface de l'implant, le procédé présentant
une première étape dans laquelle l'implant est amené dans la forme prévue et une deuxième étape dans laquelle la surface de l'implant est modifiée pour obtenir une rugosité ou une structure poreuse qui favorise l'intégration dans l'os,
la surface de l'implant n'étant modifiée que dans des premières régions d'une partie céramique de la surface de l'implant dans la deuxième étape, les deuxièmes régions de la partie céramique de la surface de l'implant n'étant pas modifiées ou ne l'étant que dans une mesure réduite,
**caractérisé en ce que**
l'implant présente des structures (7, 31) de retenue et/ou de blocage de la rotation et
**en ce que** des reliefs (11) de ces structures de retenue et/ou de blocage de la rotation sont configurés au moins en partie comme premières régions tandis que des creux (12) prévus entre ces reliefs sont configurés comme deuxièmes régions.

9. Procédé selon la revendication 8, **caractérisé en ce que** la modification de surface est réalisée par une opération d'enlèvement de matière au moyen d'un agent d'enlèvement orienté.

10. Procédé selon la revendication 9, **caractérisé en ce que** l'agent d'enlèvement orienté est un faisceau laser, la modification de la surface étant réalisée à l'aide d'un faisceau laser concentré.

11. Procédé selon la revendication 10, **caractérisé en ce que** le laser est concentré de telle sorte que la profondeur de champ soit plus petite que la hauteur des reliefs caractéristiques au niveau de la partie de la surface de l'implant.

12. Procédé selon les revendications 10 ou 11, **caractérisé en ce qu'**un déplacement relatif du faisceau laser concentré par rapport à l'implant ainsi que la puissance du faisceau laser sont commandés de telle sorte que le faisceau laser n'agisse pour enlever de la matière qu'aux emplacements qui font partie des premières régions.

13. Procédé selon les revendications 9 à 12, **caractérisé en ce que** la direction suivant laquelle l'agent agit forme avec l'axe de l'implant un angle (α) plus petit que l'angle maximum (β) de la pente par rapport à l'axe de l'implant sur les reliefs de la partie de la surface de l'implant, de telle sorte que ces reliefs projettent une ombre sur des creux situés entre les reliefs.

14. Procédé selon l'une des revendications 8 à 13, **caractérisé en ce que** dans la deuxième étape, les deuxièmes régions sont couvertes par un masque (15).
